# EUROPEAN PATENT APPLICATION

(11) **EP 4 570 236 A1**
(43) Date of publication of application: **18.06.2025**
(21) Application number: 23215866.7
(22) Date of filing: 12.12.2023
(51) Int. Cl.: A61K 8/26, A61K 8/49, A61Q 11/00, A61Q 17/00

(54) **USE OF A COMPOSITION FOR PROVIDING ANTIVIRAL PROTECTION**

(71) Applicant: Unilever IP Holdings B.V., 3013 AL Rotterdam (NL)
(72) Inventor: MUKHERJEE, Sayandip, 6708 WH Wageningen (NL); PATHAK, sandip Bhanudas, 6708 WH Wageningen (NL); SAJI, Maya Treesa, 6708 WH Wageningen (NL)
(74) Representative: Tansley, Sally Elizabeth

(57) **Abstract**

The present invention relates to the use of a composition. It is desired to provide a composition which provides prolonged protection and where the composition is physiologically acceptable to the users.

We have found that a bipolar antimicrobial particle according to the present invention or a quaternary ammonium antimicrobial agent can deliver prolonged protection against enveloped viruses and bacteria. They can be successfully incorporated into a personal care composition to provide virucidal activity. It was found that the virucidal activity persist for up to 2 hours after administration of the antimicrobial composition in the oral cavity, where it is mixed with saliva. The present inventors have also found that the bipolar antimicrobial particle can act on a virus particle present in the mucosal cavity, which virus particle has been present in the oral cavity up to 2 hours prior to the administration of the bipolar antimicrobial particle.

## Description

### Field of the Invention

The present invention relates to an antimicrobial composition, particularly the invention relates to use of an antimicrobial composition for prolonged protection against bacterial and viral infections.

### Background of the Invention

Numerous topical compositions for treating human body surfaces such as skin, lesions, or mucosal surfaces are available. Topical compositions, however, are often easily washed away, or otherwise have limited effectiveness because they are not kept in contact with the body surface for a duration long enough to maximize their effectiveness. Many topical compositions are intermittent, transitory options that do not provide sustained protection.

Most oral care compositions such as toothpastes usually contain one or more antibacterial agent for oral hygiene. Triclosan is an antibacterial agent which has found widespread acceptance. Zinc chloride, zinc citrate and other zinc salts represent another class of widely used antibacterial agents.

Cationic agents like chlorhexidine and cetyl pyridinium chloride (CPC) are believed to be more substantive and can be readily absorbed and gradually released. However, the successful formulation of a cationic antibacterial agent is a challenge at least in part due to incompatibility of cationic antibacterial agents with some of the common ingredients present in the antimicrobial composition. It is also desired to provide antimicrobial activity along with increased user acceptable.

Antimicrobial composition, particularly oral care composition is particularly designed to provide anti-bacterial benefit for preventing and treatment of gingivitis, tooth whitening, breath freshness, mitigation of tooth decay and mitigation of tooth sensitivity. However, in recent years there has been an increase in the frequency of the transmission of novel viral infections into the human population.

One such composition is disclosed in WO2022/156990 (Unilever) which is directed towards an oral care composition having a quaternary ammonium compound, a PEG hydrogenated castor oil in specific ratios for use in reducing the transmission of enveloped viruses such as coronaviruses and orthomyxoviruses.

EP2938318 B1 (Unilever, 2019) discloses a toothpaste composition for use for prolonged protection against bacteria present in the oral care cavity, the toothpaste composition comprises a bipolar antimicrobial particle whose precursor is an asymmetric 1:1 or 2:1:1 clay particle having a quaternary ammonium material as the antibacterial agent.

It is desired to provide a composition which provides prolonged protection and where the composition is physiologically acceptable to the users.

### Summary of the Invention

We have found that a bipolar antimicrobial particle according to the present invention or a quaternary ammonium antimicrobial agent can deliver prolonged protection against enveloped viruses and bacteria. They can be successfully incorporated into a personal care composition to provide virucidal activity. It was found that the virucidal activity persist for up to 2 hours after administration of the antimicrobial composition in the oral cavity, where it is mixed with saliva. The present inventors have also found that the bipolar antimicrobial particle can act on a virus particle present in the mucosal cavity, which virus particle has been present in the oral cavity up to 2 hours prior to the administration of the bipolar antimicrobial particle.

Accordingly, the present invention provides a use of a bipolar antimicrobial particle or a quaternary ammonium antimicrobial agent to kill or neutralizes 99.9% viruses, said particle comprising an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antimicrobial agent attached to the coordinating cation on one of the said external surface plane.

Accordingly, the present invention provides a use of a bipolar antimicrobial particle or a quaternary ammonium antimicrobial agent to provide long lasting protection up to 2 hours, said particle comprising an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antimicrobial agent attached to the coordinating cation on one of the said external surface plane.

Accordingly, the present invention provides a use of a bipolar antimicrobial particle or a quaternary ammonium antimicrobial agent to provide lasting protection up to 2 hours and for providing healthier and safer smile, said particle comprising an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antimicrobial agent attached to the coordinating cation on one of the said external surface plane.

A second aspect of the present invention provides a use of a particle, as defined by the first aspect of the invention, which is present in a composition, wherein the composition comprises an active ingredient.

### Detailed Description of the Invention

According to the first aspect, the present invention provides use of a bipolar antimicrobial particle or a quaternary ammonium antimicrobial agent.

According to the invention provided is use of a bipolar antimicrobial particle, quaternary ammonium antimicrobial agent or combinations thereof for providing prolonged antiviral protection, preferably upto 2 hours, wherein the particle comprises: an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antimicrobial agent attached to the coordinating cation on one of the said external surface plane.

According to the invention provided is use of a bipolar antimicrobial particle, quaternary ammonium antimicrobial agent or combinations thereof to kill or neutralize 99.9% viruses, wherein the particle comprises: an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antimicrobial agent attached to the coordinating cation on one of the said external surface plane.

According to the invention provided is use of a bipolar antimicrobial particle, quaternary ammonium antimicrobial agent or combinations thereof for providing healthier and safer smile and a prolonged antiviral protection, wherein the particle comprises: an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antibacterial agent attached to the coordinating cation on one of the said external surface plane.

According to an aspect, provided is use of a bipolar antimicrobial particle, quaternary ammonium antimicrobial agent or combinations thereof for providing prolonged antiviral protection, wherein the bipolar antimicrobial particle, quaternary ammonium antimicrobial agent or combinations thereof blocks transmission of the virus from infected person up to 2 hours from the time of infection.

Use of a bipolar antimicrobial particle, quaternary ammonium antimicrobial agent or combinations thereof on a surface for providing prolonged antiviral protection, wherein the bipolar antimicrobial particle, quaternary ammonium antimicrobial agent or combinations thereof protects the surface from infection upto 2 hours from application, preferably wherein the surface is skin or oral cavity. Preferably the bipolar antimicrobial particle, quaternary ammonium antimicrobial agent or combinations thereof on the surface remains active and neutralizes the virus particle which gain entry into the oral care cavity subsequently.

### Bipolar antimicrobial particle

According to the first aspect, the bipolar antimicrobial particle has a precursor which is an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antimicrobial agent attached to the coordinating cation on one of the said external surface plane.

### Clay particle:

Particle of 1:1 clay is particularly preferred as the precursor. Preferred 1:1 clays include kaolinite and serpentine subgroups of minerals. The species included within the kaolinite subgroup include but are not limited to kaolinite, dickite, halloysite and nacrite. The species within the serpentine subgroup include but are not limited to chrysolite, lizardite, and amesite.

Preferred 2:1:1clays include chlorite group of minerals. The chlorite comprises tetrahedral-octahedral-tetrahedral sheets like 2:1 clays, with an extra weakly bound brucite like layer between tetrahedral layers. The tetrahedral sheet preferably comprises coordinating tetrahedral cations of silicon. The tetrahedral sheet may also include isomorphously substituted coordinating tetrahedral cations which are not silicon. Isomorphously substituted coordinating tetrahedral cations include, but are not limited to, cations of aluminum, iron, or boron.

The octahedral sheet preferably has coordinating octahedral cation of aluminum. The octahedral sheet may also comprise isomorphously substituted coordinating octahedral cations which are not aluminium. Isomorphously substituted coordinating octahedral cations include cations of magnesium or iron. It is preferred that the antimicrobial agent is attached to the coordinating cations on the exterior side of one of the external surface planes. Accordingly, the antimicrobial agent is attached to coordinating cations on the exterior side of the tetrahedral sheet. Alternatively, the antimicrobial molecule is attached to the coordinating cations on the exterior side of the octahedral sheet. Coordinating cations on the exterior side of each of the tetrahedral and the octahedral surface sheets are attached to an antimicrobial molecule, with the proviso that the antimicrobial molecule attached to the coordinating cations on the exterior side of the tetrahedral surface sheet is not identical to the molecule attached to the coordinating cations on the exterior side of the octahedral surface sheet. The antimicrobial agent is preferably attached to the coordinating cations on the external surface of the octahedral surface plane and is not preferably attached to coordination cations of non-exterior tetrahedral or octahedral plane or on the interior side of the surface sheets.

Preferably the weight ratio of the clay to the quaternary ammonium antimicrobial agent in the bipolar antimicrobial particle ranges from 1:0.001 to 1:1, more preferably from 1:0.001 and 1:0.1.

It is preferred that in the antimicrobial particle, the quaternary ammonium antibacterial agent is attached to coordinating cations on the external surface of the octahedral surface plane.

Further details of the particle can be found in WO2011/036031A1 (Unilever).

Preferably the median particle size (D₅₀) of the bipolar antimicrobial particle ranges from 0.1 µm to 10 µm, more preferably 0.4 µm to 1 µm and most preferably 0.5 µm to 0.8 µm.

Particle size distribution (D₅₀) is also known as the median diameter or the medium value of the particle size distribution, it is the value of the particle diameter at 50 % in the cumulative distribution. It is an important parameter characterizing particle size. For example, if D₅₀=5.8 µm, then 50 % of the particles in the sample are larger than 5.8 µm, and 50 % smaller than 5.8 µm. D₅₀ is usually used to represent the particle size of group of particles. The D₅₀ is the size in microns that splits the distribution with half above and hlf below this diameter.

### Quaternary ammonium antimicrobial agent:

Preferably the quaternary ammonium material is selected from a quaternary ammonium material comprising a single alkyl or alkenyl long chain having an average chain length greater than or equal to C₂₀ or a quaternary ammonium material selected from cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride, domiphen bromide.

More preferably the quaternary ammonium material is selected from cetylpyridinium chloride (CPC), cetyltrimethylammonium chloride (CTAC), cetyltrimethylammonium bromide (CTAB), benzalkonium chloride (BKC), benzethonium chloride, cetrimide or quaternium.

Cetylpyridinium chloride (CPC) is particularly preferred. It is well known as an antibacterial agent especially for the inhibition of plaque.

The antibacterial activity of cetylpyridinium chloride is, without being bound to the theory, believed to be linked to the cationic charge of its amine group. Thus, cetylpyridinium chloride is attracted to and binds to negatively charged protein moieties on the cell membrane or cell wall of the microorganism and to tooth surfaces which are also typically negatively charged. The resulting attachment to microorganisms disrupts the cell wall structure causing leakage of the intracellular fluids, eventually killing the associated microorganism. However, cetylpyridinium chloride is generally not effective in many systems because of its tendency to complex with component that carry a negative charge. When bound to the clay in this manner, cetylpyridinium chloride is unavailable for effective contact with tooth surfaces and microorganisms, thereby rendering the active agent ineffective for its intended purpose.

Without being bound by theory, the mode of action of virucidal efficacy of CPC or antimicrobial particle according to the invention against enveloped viruses is majorly believed to be through disruption of viral lipid membrane or through the binding of CPC to envelope proteins leading to conformational changes in envelope which affect the receptor binding and reducing the infectivity of viruses. Overall, instant broad spectrum virucidal efficacy of quaternary ammonium compound like CPC is through lipid membrane disruption in combination with protein denaturation.

Preferred oral care composition includes 0.1 wt.% to 10 wt% bipolar antimicrobial particles, more preferably 0.5 wt% to 5 wt% particles. It is further preferred that such particle contains 1 % to 60 % loading of the antimicrobial agent. A particularly preferred antimicrobial agent is Cetylpyridinium chloride.

Preferred oral care compositions contain 0.01 to 3 wt% quaternary ammonium antimicrobial agent, more preferably 0.01 to 1 wt% quaternary ammonium antimicrobial agent. A particularly preferred quaternary ammonium antimicrobial agent is Cetylpyridinium chloride.

### Composition

According to a second aspect, the present invention provides a use of quaternary ammonium antimicrobial agent, the bipolar antimicrobial particle, or combinations thereof in a composition for providing long lasting protection against virus.

Use of a bipolar antimicrobial particle, quaternary ammonium antimicrobial agent or combinations thereof as claimed in any one of the preceding claims, wherein the particle or quaternary ammonium antimicrobial agent is present with an antimicrobial composition.

Preferably in the use according to the invention, the composition is a personal care composition, selected from a skin care composition, skin cleansing composition, and an oral care composition. Preferably the oral care composition is a toothpaste, dentifrice, tooth powder, topical oral gel, mouth rinse, denture product, mouth spray, lozenge, oral tablet, chewing gum, impregnated dental implement, dental floss, and combinations thereof.

The use of the invention is particularly effective against virus, particularly those found in personal care environments, such as oral cavity, and external skin.

Preferably the virus are those enveloped viruses belonging to Orthomyxoviruses, paramyxoviruses, Coronaviridae and Herpesviruses like Influenza virus, respiratory syncytial virus and Herpes Simplex virus.

The use according to the present invention provides long lasting protection upto 2 hours after the administration of the bipolar antimicrobial particle preferably by reduction of transmission of enveloped viruses preferably coronaviruses and orthomyxoviruses, more preferably SARS-CoV-2 (covid 19), hCoV 229E, HcoV OC43, influenza A viruses, respiratory syncytial virus & Herpes simplex virus.

The use according to the invention is a bipolar antimicrobial particle as described above for use in the control of oral bacteria, preferably *Streptococcus mutans,* ATCC 25175 / DSM 20523, Streptococcus sanguinis, ATCC 10556 / DSM 20567, *Streptococcus salivarius,* ATCC 13419 / DSM 20560 *Streptococcus mitis,* ATCC 6249, *Neisseria subflava* ATCC 49275 / DSM 17610, *Actinomyces viscosus* ATCC 15987.

The mouthwash composition is in a swishable form or spray form, mouthwashes in a swishable form being particularly preferred. The term "mouthwash" generally denotes liquid formulations which are in swishable form and used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated.

Preferably the mouthwash composition includes an aqueous base, particularly an aqueous continuous phase. The amount of water generally ranges from 70 wt.% to 99 wt.% based on the total weight of the mouthwash composition.

A mouthwash composition according to the invention will generally contain further ingredients to enhance performance and/or consumer acceptability, such as the humectants and surfactants. The amount of humectant generally ranges from 1 wt.% to 20 wt.%, more preferably from 1.5 wt. to 5 wt.% based on the total weight of the mouthwash composition. Preferred humectants that are present at the above levels include polyols, more preferred is sorbitol and/or glycerol, glycerol is particularly preferred. It is preferable if the level of ethanol in the total composition is less than 0.1 wt.%.

A mouthwash composition according to the invention may comprise a preservative, preferred preservatives are benzyl alcohol, and phenoxyethanol. Preferably the level of preservative is from 0.1 wt.% to 1 wt.% of the total composition.

The mouthwash composition of the invention may comprise a deposition aid. The term "deposition aid" in the context of this invention generally means a material which aids deposition of anti-bacterial agents from the composition. Suitable deposition aids for use in the invention will generally dissolve or disperse in water at a temperature of 25°C. Preferred deposition aids for use in the invention are water soluble. The term "water-soluble" in this particular context generally means that the deposition aid has an aqueous solubility of at least 10g/L at 25°C, and more preferably at least 30g/l at 25°C (where the solubility is determined in unbuffered distilled water). It is particularly preferable that the deposition aid remains water soluble after drying, so that it can be redissolved. This prevents undesirable build-up of the deposition aid on the teeth after repeated usage of the composition. Suitable deposition aids for use in the invention include polymeric materials, preferably polymeric materials which are water soluble as defined above.

### Surfactant:

The composition may contain low levels of surfactant based on the total weight of the composition. If present, the surfactant is preferably present at levels of less than 3 wt.% of the total composition, more preferably at levels less than 2 wt.%, most preferably at levels of less than 1.5 wt.%.

Suitable surfactants for use in the invention comprises nonionic surfactants, such as polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Preferred nonionic surfactants are ethoxylated polyethylene glycol esters of castor oil, particularly PEG 40 hydrogenated castor oil.

Other suitable surfactants include amphoteric surfactants, such as betaines or sulphobetaines. Mixtures of any of the above-described materials may also be used. Preferably the composition is free of anionic surfactants.

Compositions of the present invention may also contain further optional ingredients customary in the art such as fluoride ion sources, anti-calculus agents, buffers, flavouring agents, stability agents such as EDTA, sweetening agents, colouring agents, opacifying agents, anti-sensitivity agents and antimicrobial agents.

Use of the composition in the context of this invention typically involves application of the composition to the oral cavity, for a recommended time period before being expectorated. Preferred application times are from 10 to 60 seconds.

### Oral care composition

According to the present invention the preferred personal care composition is oral care composition, particularly the oral care composition which are suitable for brushing and/or rinsing the surface of the oral cavity.

Preferably the composition is an oral care composition. Preferably the oral care composition is a toothpaste, dentifrice, tooth powder, topical oral gel, mouth rinse, denture product, mouth spray, lozenge, oral tablet, chewing gum, impregnated dental implement, dental floss, and combinations thereof.

The composition of the invention is most preferably in the form of a dentifrice. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically, such a composition is used in conjunction with a cleaning implement such as a toothbrush, usually by applying it to the bristles of the toothbrush and then brushing the accessible surfaces of the oral cavity.

More preferably the oral care dentifrice composition has 5 wt.% to 60 wt.% abrasive, and a surfactant. Preferably the oral care composition is an oral care dentifrice composition, or a toothpaste composition.

Preferably the dentifrice/toothpaste is in the form of an extrudable semi-solid such as a cream, paste or gel (or mixture thereof).

A composition according to the invention (such as a dentifrice/toothpaste) will generally contain further ingredients to enhance performance and/or consumer acceptability, in addition to the ingredients specified above.

### Abrasive:

A toothpaste generally contains an abrasive, preferably the abrasive is a silica-based abrasive, calcium-based abrasive, or a mixture thereof. Oral care composition in the form of gel usually comprises silica-based abrasive, whereas opaque creams generally contain calcium-based abrasives, especially chalk. The quantity of the abrasive needs some control because excess abrasive causes more abrasion. Further, uncontrolled amounts of abrasives will also adversely affect viscosity of the toothpaste.

Compositions according to the invention, particularly toothpastes, preferably comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.

Preferred toothpaste compositions have 10 wt.% to 60 wt.% abrasive, more preferably 30 wt.% to 50 wt.% and most preferably 45 wt.% to 55 wt.% abrasive which may be silica-based abrasive or calcium-based abrasive.

Preferred toothpaste composition includes a calcium-based abrasive. A particularly preferred abrasive is fine ground natural chalk (FGNC). It is obtained from limestone or marble. FGNC may also be modified chemically or physically by coating during or after milling by heat treatment. Typical coating materials include magnesium stearate and oleate. The morphology of FGNC may also be modified during the milling process by using different milling techniques, for example, ball milling, air-classifier milling or spiral jet milling. FGNC can be used as the sole calcium containing abrasive. However, FGNC can also be used with other calcium containing abrasives to balance the abrasion.

Other preferred calcium-based abrasives include dicalcium phosphate (DCP), calcium pyrophosphate and precipitated calcium carbonate (PCC).

When a combination of calcium-based abrasives is used, it is preferred that FGNC is 35 to 100 %, more preferably 75 to 100 % and especially from 95 to 100 % of the total abrasive. In such cases, the balance, most preferably, is PCC.

Other abrasives can also be used depending upon the intended degree of abrasion and the composition of the toothpaste. These include synthetic abrasive polishing agents such as amorphous precipitated silica and silica gels. Other abrasives include magnesium carbonate, sodium metaphosphate, potassium metaphosphate, zirconium silicate, potassium metaphosphate, magnesium orthophosphate, tricalcium phosphate, magnesium orthophosphate, tri-magnesium phosphate, aluminum silicate, zirconium silicate and perlite.

Preferably the composition, particularly a toothpaste, comprises a silica-based abrasive. The preferred abrasive silicas used in the present invention is a silica with a low refractive index. It may be used as the sole abrasive silica, or in conjunction with a low level of other abrasive silicas, e.g., those according to EP 236070. Typical examples of suitable low refractive index abrasive silicas (e.g., having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the total composition generally ranges from 5-60% by weight, usually 5 wt.% to 20 wt.%.

### Surfactant:

Preferably the toothpaste composition includes a surfactant, also commonly referred to as sudsing agent. Suitable surfactants are those which are reasonably stable and provide foam throughout a wider pH range. Preferably the surfactant may be selected from the group consisting of anionic surfactant, amphoteric surfactant, nonionic surfactant, or mixtures thereof.

Preferably the surfactant is anionic. Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate and sodium coconut monoglyceride sulfonates are examples of anionic surfactants of this type.

Preferred compositions contain 0.25 wt.% to 12 wt.%, more preferably from 0.5 wt.% to 8 wt.%, and most preferably from 1 wt.% to about 6 wt.% anionic surfactants.

Some anionic surfactants, in particular, sodium lauryl sulphate itself have antibacterial effect. Such action provides some degree of instant antibacterial effect. However, this effect is generally very short-lived. Therefore, the more sustainable antibacterial action is provided by the antibacterial agent such as triclosan. Some of the tests which are routinely performed to quantify the antibacterial action show inconsistent results because the procedure does not distinguish between the effect of the anionic surfactant and that of the antibacterial action. However, single species assay can provide more accurate results because the test concentrates only on the effect of the antibacterial agent.

Other surfactants like nonionic, amphoteric or zwitterionic surfactants may also be included.

Nonionic surfactants can be broadly defined as compounds produced by the condensation of alkylene oxide groups (hydrophilic in nature) with an organic hydrophobic compound which may be aliphatic or alkyl- aromatic in nature. Examples of suitable nonionic surfactants include poloxamers (sold under trade name PLURONIC^{®}), polyoxyethylene, polyoxyethylene sorbitan esters (sold under trade name TWEENS^{®}), POLYOXYL^{®} 40 hydrogenated castor oil, fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and mixtures of such materials.

Useful amphoteric surfactants can be broadly described as derivatives of aliphatic secondary and tertiary amines in which the aliphatic radical is a straight chain or branched and wherein one of the aliphatic substituent contains from about 8 to about 18 carbon atoms and one contains an anionic water-solubilizing group, e.g., carboxylate, sulfonate, sulfate, phosphate, or phosphonate. Other suitable amphoteric surfactants are betaines, specifically cocamidopropyl betaine. Mixtures of amphoteric surfactants can also be used.

### Bipolar antimicrobial particle:

Preferred oral care compositions comprise bipolar antimicrobial particle in an amount ranging from 0.1 wt.% to 10 wt.%, more preferably 0.5 wt.% to 5 wt.% particles. It is further preferred that such particle contains 1 wt.% to 60 wt.% loading of the quaternary ammonium antimicrobial agent. A particularly preferred quaternary ammonium antibacterial agent is cetylpyridinium chloride.

### Thickening silica:

Oral care composition preferably includes a thickening silica, preferably in an amount ranging from 0 to 2 wt% thickening silica by weight of the oral care composition, Conventional gel toothpastes generally contain upto 8.5 wt% thickening silica whereas opaque toothpastes typically contain 4 wt.% to 10 wt.% thickening silica. The toothpaste compositions can include up to 2 wt.% thickening silica. Preferred compositions have 1.5 wt.%, or even less than 1 wt.% thickening silica. Optimal compositions have less than 0.5 wt.% thickening silica. Highly preferred compositions do not contain thickening silica.

When present, preferred thickening silicas include AEROSIL^{®} T series from Degussa or the CAB-O-SIL^{®} series from Cabot Corporation, silica gels such as the SYLODENT^{®} or SYLOX^{®} series from W. R.Grace & Co or precipitated silica such as ZEOTHIX^{®} 265 from J. M. Huber Corporation. Useful silica thickeners also include ZEODENT^{®} 165, ZEODENT^{®} 163 and/or 167 and ZEOFREE^{®} 153, 177, and/or 265 silicas, all available from J. M. Huber Corporation. Other preferred thickening silicas include MFIL^{®}, MFIL^{®}-P (From Madhu Silica), SIDENT^{®} 22 S and AEROSIL^{®} 200 (Ex. Evonik Industries), SYLODENT^{®} and PERKASILO thickening silicas from WR Grace & Company and Tixosil^{®} 43 and 331 from Rhodia, synthetic finely divided pyrogenic silica such as those sold under the trademarks SYLOID^{®} 244, SYLOID^{®} 266 and AEROSIL^{®} D-200.

### Humectant:

Oral care composition preferably includes a humectant. Humectants are generally included in toothpastes for a soft and supple mouth feel. Humectants also reduce the tendency of toothpastes to lose moisture. Oral care compositions, preferably include from 3.5 wt.% to 40 wt.% humectants. Further preferred compositions have 10 wt.% to 40 wt.%, more particularly 10 wt.% to 20 wt.% humectants.

A particularly preferred humectant is sorbitol, generally available as 70% aqueous solution.

Other preferred humectants include glycerine, maltitol and xylitol. More preferred toothpastes contain glycerine and sorbitol for a lubricated mouth feel, but their cumulative levels should not exceed the disclosed upper limit.

### Binder:

Oral care composition preferably includes a binder. Preferred toothpaste compositions have a binder, which lends a good structure to the paste. Cellulosic binders are especially preferred. Preferred cellulosic binders include cellulose ethers, which include hydroxyethyl cellulose (HEC), hydroxypropyl cellulose (HPC), ethylhydroxyethyl cellulose (EHEC), carboxymethyl cellulose (CMC), carboxymethylhydroxyethyl cellulose (CMHEC), hydroxypropylhydroxyethyl cellulose (HPHEC), methyl cellulose (MC), methylhydroxypropyl cellulose (MHPC), methylhydroxyethyl cellulose (MHEC), carboxymethylmethyl cellulose (CMMC), hydrophobically modified carboxymethyl cellulose (HMCMC), hydrophobically modified hydroxyethyl cellulose (HMHEC), hydrophobically modified hydroxypropyl cellulose (HMHPC), hydrophobically modified ethylhydroxyethyl cellulose (HMEHEC), hydrophobically modified carboxymethylhydroxyethyl cellulose (HMCMHEC), hydrophobically modified hydroxypropylhydroxyethyl cellulose (HMHPHEC), hydrophobically modified methyl cellulose (HMMC), hydrophobically modified methylhydroxypropyl cellulose (HMMHPC), hydrophobically modified methylhydroxyethyl cellulose (HMMHEC), and hydrophobically modified carboxymethylmethyl cellulose (HMCMMC).

Other cellulosic binders include cationic hydroxyethyl cellulose (cationic HEC), cationic hydrophobically modified hydroxyethyl cellulose (cationic HMHEC) and microcrystalline cellulose.

A highly preferred binder is Sodium carboxymethyl cellulose (SCMC). Particularly preferred sodium carboxymethyl celluloses include those with degree of substitution of from 0.6 to 0.99, preferably from 0.7 to 0.95.

In addition to, or as a replacement of cellulosic binder, guar gum or its derivatives could be used, however, such binders are less preferred to the cellulosic ones. Such derivatives include carboxymethyl guar (CM guar), hydroxyethyl guar (HE guar), hydroxypropyl guar (HP guar), carboxymethylhydroxypropyl guar (CMHP guar), cationic guar, hydrophobically modified guar (HM guar), hydrophobically modified carboxymethyl guar (HMCM guar), hydrophobically modified hydroxyethyl guar (HMHE guar), hydrophobically modified hydroxypropyl guar (HMHP guar), cationic hydrophobically modified hydroxypropyl guar (cationic HMHP guar), hydrophobically modified carboxymethylhydroxypropyl guar (HMCMHP guar) and hydrophobically modified cationic guar (HM cationic guar).

Other less preferred gums include xanthan gum, carrageenan and derivatives, such as Irish moss and viscarin, gellan gum, sclerotium gum and derivatives, pullulan, rhamsan gum, welan gum, konjac, curdlan, algin, alginic acid, alginates and derivatives, starch phosphate derivatives, agar and derivatives, gum arabic and derivatives, pectin and derivatives, chitosan and derivatives, karaya gum, locust bean gum, natto gum, tragacanth gum, chitin derivatives, gelatin, betaglucan, dextrin, dextran, cyclodextrin and polyquaterniums, furcellaren gum, ghatti gum, psyllium gum, quince gum, tamarind gum, larch gum, and tara gum.

### Other thickening agents:

Preferred toothpaste compositions may also include one or more other thickening agents such as carboxyvinyl polymers which include carbomers which are commercially available from B. F. Goodrich as the CARBOPOL^{®} series, including CARBOPOL^{®} 934, 940, 941 and 956. Other preferred grades include acrylates/C₁₀-₃₀ alkyl acrylate crosspolymer which are commercially available as ULTREZ^{®} 21, PEMULEN^{®} TR-1, and PEMULEN^{®} TR-2, from Noveon Corporation. Preferred compositions can include 0.05 to 10 wt%, more preferably 0.1 to 5 wt%, and even more preferably 0.25 to about 4 wt% of other thickening agents.

### De-sensitizing agents:

A de-sensitizing agent is a potassium salt selected from potassium nitrate, potassium chloride, potassium citrate, potassium tartarate and potassium acetate used preferably from 0.5 to 3 wt%, more preferably from 1 to 2.5 wt % and especially from 1.7 to 2.2 wt%.

### Silicate:

Preferred toothpaste compositions can have alkali metal silicate. The alkali metal is sodium or potassium, preferably sodium. Sodium silicate is generally available as 10 to 40 % aqueous solution, most common being 30 % solution. Sodium silicate is available as neutral sodium silicate or alkaline sodium silicate. Preferred toothpastes have neutral sodium silicate. Sodium silicate is available with varying ratios of Na₂O: SiOz.

Sodium silicate with Na₂O: SiOz ratio in the range of 3.0 to 3.8 is preferred, more highly preferred range being 3.25 to 3.5. Preferred toothpastes include 0.1 to 5 wt% silicate (on dry weight basis). Thus, a 30 % solution of sodium silicate is added to the composition in an amount in the range of 0.3 to 16 wt%.

### Anti-caries agent:

Preferred compositions can include one or more anti-caries agent. Such agents are typically fluorides. It is preferred that the source of fluoride is an alkali-metal salt of monofluorophosphoric acid, preferably sodium monofluorophosphate (SMFP).

SMFP is the fluoride source of choice when it comes to toothpaste compositions having Calcium based abrasives, especially chalk as sodium fluoride reacts with the calcium carbonate to form insoluble calcium fluoride which has limited anti-caries activity. Preferred compositions include 0.01 to 2 wt%, more preferably 0.15 to 1 wt% and especially preferably 0.2 to 0.5 wt% anti-caries agent. It is preferable to maintain the free fluoride ion concentration from 100 to 2000 ppm, preferably from 900 to 1500 ppm. Other preferred anti-caries agents include sodium- and stannous fluoride, aminefluorides, sodium trimetaphosphate and casein.

### Other antibacterial agents:

Additional anti-bacterial agents can be present in the composition, though not strictly necessary. Examples include triclosan and other halogenated bisphenolic compounds such as 2,2' methylenebis-(4-chloro-6-bromophenol).

### Preservative:

Toothpastes with calcium containing abrasives especially chalk are prone to bacterial growth. Certain preservatives, e.g., methyl, ethyl, butyl, propyl, and isopropyl esters of parahydroxybenzoic acid may be particularly useful against bacterial growth. A mixture of methyl, ethyl, butyl, and propyl esters of parahydroxybenzoic acid is particularly preferred.

The activity of this mixture can be enhanced by adding phenoxyethanol. Formaldehyde and dimethyl hydantoin are other preferred preservatives. Preservatives are generally included at 0.05 to 0.8 wt%.

### Antioxidant:

Preferred antioxidants are those which are compatible with other components and are not hazardous to health. These include ascorbic acid, ascorbyl palmitate, thiodipropionic acid, calcium ascorbate, dilauryldithiopropionate, gum guaiac, sodium ascorbate, butylated hydroxyl toluene, butylated hydroxyl anisole, and tocopherols. Mixture of antioxidants can be used.

When present, the antioxidant is added in a level effective to reduce or mitigate discoloration that would otherwise result from oxidation of the components of the toothpastes. Preferred levels are from 0.01 to 1 wt%.

### Sweetening agent:

Toothpastes may also contain a sweetening agent. Preferred sweetening agents include sodium saccharin, aspartame, sucralose, thaumatin, acesulfame potassium, stevioside, stevia extract, paramethoxy cinnamic aldehyde, neohesperidyl dihydrochalcone and perillartine. Typical levels are from 0.005 to 5 wt%, more preferably from 0.01 to 1 wt%.

### Bleaching agent:

Preferred toothpastes can include one or more bleaching agents such as peroxy compounds e.g., potassium peroxydiphosphate, effervescing systems such as sodium bicarbonate/citric acid systems, colour change systems, and chelating agents from 0.001 to 6 wt%, preferably at from 0.5 to 4 wt%, which include alkali metal salts of citric acid, alanine, glycine, and serine.

The most preferred are the alkali metal salts of citric acid, especially potassium citrate and most preferably tri-potassium citrate.

Liposomes can be used to improve delivery or stability of one or more active ingredients. Preferred compositions may also include one or more of breath strips, sparkles, large silica particles, granules, beads, and flavour encapsulates for enhanced sensory benefits or for visual appeal.

Titanium dioxide can also be added to the composition for opacity. Titanium dioxide is generally included from 0.25 to 5 wt%.

It is preferred that the compositions contain a flavour. Suitable flavoring components include oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, ethyl vanillin, heliotropine, 4-cis-heptenal, diacetyl, methyl-para-tert-butyl phenyl acetate, and mixtures thereof. Coolants may also be part of the flavor system. Preferred coolants are the paramenthane carboxyamide agents such as N-ethyl-p-menthan-3-carboxamide (known commercially as "WS-3^{®}") and mixtures thereof. The flavour is generally from 0.001 to 5 wt%.

### Mouthwash composition

According to the present invention the preferred personal care composition is oral care composition, particularly where the oral care composition is a mouthwash composition.

Preferably the oral care composition is a mouthwash composition comprising:
i. a PEG hydrogentated castor oil; and
ii. a bipolar antimicrobial particle, a quaternary ammonium antimicrobial agent or a combination thereof;
wherein the bipolar antimicrobial particle and the PEG hydrogentated castor oil are present as a co-micelle and in which the weight ratio of the PEG hydrogentated castor oil to the bipolar antimicrobial particle ranges from 19:1 to 10:1.

### PEG hydrogenated castor oil

Mouthwash composition according to invention comprise PEG hydrogenated castor oil, preferably PEG40 hydrogenated castor oil.

Preferably the level of PEG hydrogenated castor oil, preferably PEG40 castor oils ranges from 0.6 wt.% to 1.4 wt.% of the total composition, more preferably from 0.7 wt.% to 1.2 wt.% most preferably from 0.8wt.% to 1wt.%.

Preferably the weight ratio of PEG hydrogenated castor oil to quaternary ammonium compound is preferably from 8:1 to 10:1, more preferably 0.8 1 to 5:1 most preferably 0.8:1 to 2:1 It is particularly advantageous if the weight ratio of PEG hydrogenated castor oil to quaternary ammonium is from 0.9:1 to 1.5.1 most preferably 1.1:1.

### Sanitizing composition

According to the present invention the preferred personal care composition is skin care composition, particularly a sanitizing composition.

Preferably the sanitizing composition is a sanitizing precursor comprising:
i. less than 15% by weight water; and
ii. a bipolar antimicrobial particle, a quaternary ammonium antimicrobial agent or combinations thereof;
wherein the sanitizing precursor is suitable to be diluted to produce a sanitizing composition comprising at least 80% by weight water.

Preferably the sanitizing composition is capable of delivering a bacteria log kill of at least 2 and a viral log inactivation of at least 2 in less than 3 minutes after topical application. Preferably the sanitizing composition is substantially free of ethanol.

Preferably the sanitizing composition includes a total surfactant content more than 4.5 wt.% by weight and preferably a cationic surfactant is present in the composition in addition to the bipolar antimicrobial particle, quaternary ammonium antimicrobial agent and mixtures thereof. Preferably the cationic surfactant in the composition includes a combination of both cetrimonium chloride and benzalkonium chloride, and preferably benzalkonium chloride makes up at least 16.5% by weight of the combination.

Preferably the sanitizing composition according to the invention includes a
a) at least 80% by weight water;
b) bipolar antimicrobial particle, quaternary ammonium antimicrobial agent or combination thereof; and,
c) a cationic surfactant.

Preferably the sanitizing composition is capable of delivering a bacteria log kill of at least 2 and a viral log inactivation of at least 2 in less than 3 minutes after topical application and further preferably the composition is substantially free of ethanol. Preferably when total surfactant in the composition is more than 4.5% by weight and preferably when cationic surfactant in the composition includes a combination of both cetrimonium chloride and benzalkonium chloride, benzalkonium chloride makes up at least 16.5% by weight of the combination.

Skin, as used herein, is meant to include skin on the arms (including underarms), face, feet, neck, chest, hands, legs, buttocks, and scalp (including hair).

Sanitizing as used herein means a bacteria log kill of at least 2 and a viral log inactivation of at least 2 (both achieved) in less than 3 minutes after topical application to a surface.

Sanitizing precursor means a composition suitable to be diluted (or hydrated) with water to produce a sanitizing composition ready for use.

In a preferred embodiment, the composition of the present invention is a topical composition to apply to skin for sanitizing by significantly reducing the amount of bacteria and viruses on the skin. The composition of the present invention may optionally comprise skin benefit ingredients added thereto such as emollients, vitamins and/or derivatives thereof, resorcinols, retinoic acid precursors, colorants, moisturizers or humectants, fragrances, sunscreens, a mixture thereof or the like. The skin benefit ingredients may be water or oil soluble. The sanitizing composition, therefore, is an aqueous based composition with a pH from 3.0 to 8.2, and the composition is water continuous. Viscosity, as used herein, is taken with a Brookfield RV spindle 6 at 20 rpm for 30 seconds at 25°C unless noted otherwise. In still another embodiment, the composition of this invention is a non-therapeutic and non-medicinal composition which is a water continuous liquid having a viscosity under 30,000 cps. In the absence of explicitly stating otherwise, all ranges described herein are meant to include all ranges subsumed therein. As used herein, substantially free of means less than 10% by weight.

Water will typically make up at least 80% by weight of the sanitizing composition, and preferably, at least 82% by weight of the composition, and most preferably, at least 84% by weight of the sanitizing composition. In an embodiment of the invention, water makes up from 85 to 99.5%, and preferably, from 87 to 98.8%, and most preferably, from 89 to 97% by weight of the composition, including all ranges subsumed therein.

The sanitizing composition of the present invention is substantially free of ethanol (i.e., less than 10% by weight), and preferably, comprises less than 6% by weight ethanol, and most preferably, from 2.5 to 5.5% by weight ethanol. In an embodiment of the invention, the composition of the present invention may have less than 1% by weight ethanol. In another desired embodiment, the composition may have no (0.0% by weight) ethanol. Additionally, and excluding ethanol and not including diols and polyols, the composition of the present invention will comprise less than 8%, and preferably, less than 6.5%, and most preferably, less than 5% by weight aliphatic alcohol. In yet another embodiment of the invention, the sanitizing composition of the present invention may comprise from 0.001 to 2.5% by weight aliphatic alcohol or no (0.0% by weight) aliphatic alcohol.

It is within the scope of this invention to include optional surfactants along with the cationic surfactants described herein. The optional surfactants can include those which are amphoteric (which depending on pH can be zwitterionic). Illustrative optional amphoteric surfactants suitable for use generally include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants that may be used include sodium lauroamphoacetate, sodium cocoamphoacetate, sodium lauroamphoacetate, sodium cocoamphoacetate a mixture thereof or the like.

Illustrative examples of the zwitterionic surfactants suitable for optional use include betaines like cocodimethyl carboxymethyl betaine, cocoamidopropyl betaine and laurylamidopropyl betaine. An additional zwitterionic surfactant suitable for use includes cocoamidopropyl sultaine. Such surfactants are made commercially available from suppliers like Stepan Company, and it is within the scope of the invention to optionally employ mixtures of the same.

Nonionic surfactants that may optionally be used in the sanitizing composition of the present invention include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides or the like.

The pH of the sanitizing composition of the present invention is typically from 3.0 to 8.2, and preferably, from 3.1 to 7.5, and most preferably, from 3.2 to 7.0. In an embodiment of the invention, the pH of the sanitizing composition is from 3.3 to 6.5, and preferably, from 3.3 to 5.5. Adjusters suitable to modify the pH of the sanitizing composition of this invention may be used. Such pH adjusters include triethylamine, NaOH, KOH, H₂SO4, HCl, C₆H₈O₇ (i.e., citric acid) or a mixture thereof. Buffers, like potassium oxide, to stabilize pH are also suitable for use. The pH of the composition is assessed by using conventional instrumentation such as a pH meter made commercially available from Thermo Scientific^{®}.

As to optional skin benefit agents, these can be water and/or oil soluble and are preferably water soluble. Humectants, like water soluble polyols, are generally desired. These include sorbitol, glycerol (or glycerine), mannitol, xylitol, maltitol or a mixture thereof. Other humectants include propylene glycol, dipropylene glycol, polypropylene glycol (e.g., PPG-9), polyethylene glycol, hydroxypropyl sorbitol, hexylene glycol, 1,3-butylene glycol, 1 ,2-octane diol, 1,2-hexane diol, isoprene glycol, 1 ,2,6-hexanetriol, ethoxylated glycerol, propoxylated glycerol and mixtures thereof. Most preferred is glycerin, propylene glycol or a mixture thereof. In an embodiment of the invention, the polyol used is at least 50% by weight glycerol, based on total weight of the polyol used in the sanitizing composition. In another embodiment of the invention, the polyol used is a mixture of glycerol and hexylene glycol, at a weight ratio of 1:5 to 5:1 ratio, and preferably, from 4:1 to 1:4, and most preferably, from 3:1 to 1:3, including all ratios subsumed therein.

Water soluble moisturizing agents are suitable for optional use. Most preferred for optional use is hydroxyethyl urea. The same is available as a 50% aqueous liquid from Nouryon under the trademark Hydrovance^{®}.

In an often desired embodiment, the sanitizing composition of the present invention does comprise from 0.5 to 10%, and preferably, from 0.6 to 7%, and most preferably, from 0.8 to 3.5% by weight niacinamide (for longer lasting antimicrobial benefit) based on total weight of the sanitizing composition. In still another embodiment, niacinamide is present from 0.7 to 1.4% by weight of the composition.

Film forming agents may be used in the compositions of the present invention. While optional, such agents can aid with composition adhering to the surface they are applied to. Film forming agents include those having hydrophilic properties and they include materials comprising polyvinylpyrrolidone (PVP), acrylates, acrylamides, and copolymers thereof. Deposition agents like organosiloxanes may also be used. When used, such agents make up from 0.001 to 1% by weight of the sanitizing composition.

Preservatives may be used in the sanitizing composition of the present invention. When sanitizing precursor is prepared and diluted with water, for example on demand, preservatives could be included but they are not required since the sanitizing composition would be used shortly after preparation. If, however, precursor is diluted with water and the resulting sanitizing composition is filled into a refill package (such as a sustainable refill spray bottle) preservative is typically recommended. Precursor will typically have less than 15% by weight water, and preferably, less than 10% by weight water, and most preferably, from 1 to 7% by weight water, based on total weight of the sanitizing precursor and including all ranges subsumed therein.

Fragrances, fixatives, opacifiers (like titanium dioxide), chelators (like EDTA) may optionally be included in the sanitizing composition of the present invention. Each of these substances may range from about 0.03 to 3%, preferably, between 0.1 and 2.6% by weight of the total weight of the sanitizing composition, including all ranges subsumed therein.

The sanitizing composition of this invention can be provided as a micellar water (surfactant forming micelles in water, preferably cationic surfactant) that surprisingly provides excellent prolonged antimicrobial and viral activity reduction upon contact with a surface. When making the precursor or sanitizing composition of the invention, ingredients are mixed with moderate shear, under atmospheric conditions and at a temperature from 20 to 65°C. Depending on the desired volume, precursor (with less than 15% by weight water) should be mixed with water followed by moderate agitation to produce desired sanitizing composition. Preferably the micellar water includes quaternary ammonium antimicrobial agent, still preferably the quaternary ammonium antimicrobial agent is Cetylpyridium chloride.

Typically, the viscosity of the sanitizing composition will be under 30,000 cps. Often the viscosity of the sanitizing composition will be from 0 to 25,000, and preferably, from 0 to 18,000, and most preferably, from 0 to 14,000 cps, including all ranges subsumed therein. In an embodiment of the invention, the viscosity of the sanitizing composition is from 1 to 3,000 cps, and in still another embodiment of the invention, the viscosity of the sanitizing composition is from 1 to 2,000 cps. In yet another embodiment of the invention, the viscosity of the sanitizing composition is from 1 to 250 cps.

To adjust sanitizing composition viscosity, it is within the scope of the invention to optionally include thickening agents. Optionally suitable for use in sanitizing composition of the present invention are thickeners classified as polysaccharides. Examples include fibers, starches, natural/synthetic gums and cellulosics.

Synthetic polymers are yet another class of effective thickening agent. This category includes crosslinked polyacrylates such as the Carbomers, acrylate copolymers, acrylates/ acrylate (C₁₀-C₃₀) alkyl acrylate crosspolymers, polyacrylamides such as Sepigel^{®} 305 and taurate copolymers such as Simulgel^{®} EG and Aristoflex^{®} AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used.

The amounts of the thickening agent, when used, may range from 0.001 to 5%, by weight of the sanitizing composition. Maltodextrin, xanthan gum, and carboxymethyl cellulose are the preferred thickening agents. Other thickening agents often desired are dilinoleyl/dimethyl carbonate copolymer, stearyl alkonium hectorite, cetyl (cetearyl, stearyl) fatty alcohols, tara gum, pentaerythrityl tetrastearate or a mixture thereof.

As to packaging, the sanitizing composition of the present invention, can be packaged in a spray bottle, or provided as an impregnating wetting agent on cotton swab, wipe, towelette, cosmetic substrate sheet (like those described in US 6,294,182 B1) or the like. As the viscosity is increased with thickening agent, the sanitizing composition gels and may be provided to consumers in a squeeze bottle as a gel composition. The spray bottle may also be metal and the sanitizing composition may be provided via conventional aerosol packaging technologies and including those which utilize air-in-bag discharging cannisters, mechanisms and actuators. It is also within the scope of the invention to include foaming agents (e.g., zwitterionic and/or amphoteric surfactants) so that the sanitizing composition can be discharged as a foam. In an embodiment of the invention, the sanitizing composition is provided in a standard spray bottle with a spray actuator and sprayed on to the surface needing sanitizing, especially skin. The sanitizing composition of the invention is particularly useful in preventing adverse skin reactions (like acne development) caused by microbes that collect under personal protective equipment (including pants, gowns, and especially, protective face masks). Thus, it is advisable to apply the sanitizing composition of the present invention to skin prior to putting on personal protective equipment (and even throughout the day when personal protective equipment is being worn).

The sanitizing composition of the invention should be supplied with instructions to apply (preferably spray) the composition on to a surface, like skin, for bacteria kill and viral activity reduction. Precursor will preferably be provided for in biodegradable packaging and the packaging used is preferably refillable or reusable, biodegradable, and/or at least 50%, and preferably, at least 100% made from post-consumer recycled resin.

The invention is further illustrated with reference to the following, non-limiting Examples. Examples according to the invention are illustrated by a number, comparative Examples are illustrated by a letter.

### Examples

Example 1: Composition according to the present invention and the use of the composition for providing prolonged antiviral benefits were prepared having the formulation as shown in the table below.

**Table 1: Mouthwash composition**

| **Ingredient** | **Mouth wash composition (wt.%)** |
|---|---|
| Glycerol | 0.5 |
| Sorbitol (70% aqueous solution) | 12.0 |
| Sodium fluoride | 0.05 |
| Cetylpyridinium chloride | 0.07 |
| Citric acid | To pH 5 |
| Benzyl alcohol | 0.4 |
| Phenoxyethanol | 0.4 |
| PEG-40 hydrogenated castor oil | 0.5 |
| Flavour | 0.2 |
| Water & minors | To 100 |

**Table 2: Toothpaste composition**

| **Ingredients** | **Toothpaste composition (wt.%)** |
|---|---|
| Sorbitol (70% aqueous solution) | 20 |
| Fine ground natural chalk | 40 |
| Hydrated silica | 3.5 |
| CPC-Clay | 1.5 |
| Cellulose Gum | 0.625 |
| PAS surfactant | 7.58 |
| Benzyl alcohol | 0.5 |
| Sodium saccharin | 0.37 |
| Sodium Mono-fluorophosphate | 1.12 |
| Sodium silicate | 1.5 |
| Tri potassium citrate monohydrate | 0.5 |
| Water & minors | To 100 |

**Table 3: Sanitizing composition**

| **Ingredients** | **Sanitizing composition (wt.%)** |
|---|---|
| Ethyl alcohol | 70 |
| Cetylpyridinium chloride | 0.05 to 1.0 |
| Propylene glycol | 5 |
| Carbopol | 1.5 |
| Perfume | 0.2 |
| Water | To 100 |

### Example 2: Evaluation of the antiviral protection of the composition.

The prolonged virucidal efficacy of CPC-containing mouthwash against Influenza A virus (IAV) and Herpes Simplex virus (HSV) (representative viruses which are relevant Human to Human transmission) were studied using in-vitro method. In a first scenario, prolonged antiviral benefits were studied to determine the protection of the healthy individual from infectious virus even after 2 hours post usage of CPC containing mouthwash. In scenario 2, the impact of virus transmission from infected individual to healthy was studied by evaluating the effect on viral load after using the mouthwash.

Testing protocol in scenario 01: Incubation of Mouthwash with saliva followed by inoculation with virus for 30 seconds.

800 µL of mouthwash solution was mixed with 100µL of either artificial saliva or pooled Human saliva and were mixed thoroughly by vortexing and incubated for 0 min, 30 min and 120 min at room temperature. Post incubation, 100µL of respective virus suspension was mixed and incubated for 30s. After incubation, 9 mL of D/E was mixed with reaction and 100µL of reaction mixture was passed through Microspin S-400 HR columns and centrifuged for 2100 rpm @ 4°C. The filtrate was then serially diluted in 2% MEM (-1 to -5, serial 10-fold dilutions) and 100µL of diluted reaction from each dilution was added on to susceptible cell monolayer. The plates were incubated @ 37°C for 5 to 7 days. The data was recorded and provided in table 4 below.

**Table 4A: Efficacy against Herpes Simplex virus**

| **Sample details** | **Log TCID₅₀ titre/ml** |
|---|---|
| Herpes Simplex virus (HSV) control | 7.00 ± 0.14 |
| Mouthwash + Saliva, 0 min | 5.00 ± 0.28 |
| Mouthwash + Saliva, 30 min | 5.50 ± 0.71 |
| Mouthwash + Saliva, 120 min | 4.80 ± 0.28 |

**Table 4B: Efficacy against Influenza A virus**

| **Sample details** | **Log TCID₅₀ titre/ml** |
|---|---|
| Influenza A virus (IAV) control | 7.00 ± 0.28 |
| Mouthwash + Saliva, 0 min | 4.50 ± 0.42 |
| Mouthwash + Saliva, 30 min | 4.80 ± 0.0 |
| Mouthwash + Saliva, 120 min | 4.70 ± 0.14 |

The data provided in table 4 (A and B) indicates significant virucidal efficacy of CPC containing mouthwash when incubated with both artificial saliva and pooled human saliva and this benefit was observed even after 2 hours incubation. There was a slight drop in virucidal efficacy especially at 2-hour time point and similar trend was observed with both artificial saliva & pooled Human saliva studies. Overall, the data indicates that CPC containing mouthwash retains the virucidal efficacy even in presence of interfering substances like saliva for up to 2 hours.

Testing protocol in scenario 02: Incubation of Virus with saliva followed by addition of the mouthwash for 30 seconds.

100µL of viral suspension was mixed with 100µL of either artificial saliva or pooled Human saliva, mixed thoroughly by vortexing and incubated for 0 min, 30 min and 120 min at room temperature. Post incubation, 800µL of CPC (quaternary ammonium antimicrobial containing mouthwash was mixed and incubated for 30 seconds. After incubation, 9 mL of D/E was mixed with reaction and 100µL of reaction mix was passed through Microspin S-400 HR columns and centrifuged for 2100 rpm @ 4°C. The filtrate was then serially diluted in 2% MEM and 100µL of diluted reaction was added on to susceptible cell monolayer. The plates were incubated @ 37°C for 5 to 7 days.

**Table 5A: Efficacy against Herpes Simplex virus**

| **Sample details** | **Log TCID₅₀ titre/ml** |
|---|---|
| Herpes Simplex virus (HSV) control, 0 min | 7.13 ± 0.11 |
| HSV + Saliva, 0 min | 4.80 ± 0.0 |
| HSV control, 30 min | 6.90 ± 0.14 |
| HSV + Saliva, 30 min | 4.80 ± 0.0 |
| HSV control, 120 min | 6.90 ±0.14 |
| HSV + Saliva, 120 min | 4.80 ± 0.00 |

**Table 5B: Efficacy against Influenza A virus**

| **Sample details** | **Log TCID₅₀ titre/ml** |
|---|---|
| Influenza A virus (IAV) control, 0 min | 7.10 ± 0.14 |
| IAV + Saliva, 0 min | 3.90 ± 0.14 |
| IAV control, 30 min | 6.60 ± 0.0 |
| IAV + Saliva, 30 min | 3.80 ± 0.0 |
| IAV control, 120 min | 6.10 ± 0.14 |
| IAV + Saliva, 120 min | 3.90 ± 0.14 |

The data provided in table 5 (A and B), indicates that the virucidal efficacy for all time points evaluated was significant indicating there was no inhibition when virus was preincubated with both saliva compositions. This indicates that there is no possible interference/interaction of salivary components to active CPC in the mouthwash. The trend was similar to both Influenza A virus and HSV virus where we observed the CPC containing mouthwash composition retained desired virucidal efficacy for up to 2 hours.

## Claims

1. Use of a bipolar antimicrobial particle, quaternary ammonium antimicrobial agent or combinations thereof for providing prolonged antiviral protection, preferably upto 2 hours, wherein the particle comprises: an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antimicrobial agent attached to the coordinating cation on one of the said external surface plane.

2. Use of a bipolar antimicrobial particle, quaternary ammonium antimicrobial agent or combinations thereof to kill or neutralize 99.9% viruses, wherein the particle comprises: an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antimicrobial agent attached to the coordinating cation on one of the said external surface plane.

3. Use of a bipolar antimicrobial particle, quaternary ammonium antimicrobial agent or combinations thereof for providing healthier and safer smile and a prolonged antiviral protection, wherein the particle comprises: an asymmetric 1:1 or 2:1:1 clay particle comprising alternating tetrahedral and octahedral sheets terminating with a tetrahedral sheet at one external surface plane and an octahedral sheet at another external surface plane with a quaternary ammonium antibacterial agent attached to the coordinating cation on one of the said external surface plane.

4. Use of a bipolar antimicrobial particle, quaternary ammonium antimicrobial agent or combinations thereof for providing prolonged antiviral protection, wherein the bipolar antimicrobial particle, quaternary ammonium antimicrobial agent or combinations thereof blocks transmission of the virus from infected person up to 2 hours from the time of infection.

5. Use of a bipolar antimicrobial particle, quaternary ammonium antimicrobial agent or combinations thereof on a surface for providing prolonged antiviral protection, wherein the bipolar antimicrobial particle, quaternary ammonium antimicrobial agent or combinations thereof protects the surface from infection upto 2 hours from application, preferably wherein the surface is skin or oral cavity.

6. Use as claimed in any one of the preceding claims, wherein the particle or quaternary ammonium antimicrobial agent or combinations thereof is present with an antimicrobial composition.

7. Use as claimed in claim 6, wherein the composition is a personal care composition, selected from a skin care composition, skin cleansing composition, and an oral care composition.

8. Use as claimed in claim 7, wherein the oral care composition is a toothpaste, dentifrice, tooth powder, topical oral gel, mouth rinse, denture product, mouth spray, lozenge, oral tablet, chewing gum, impregnated dental implement, dental floss, and combinations thereof.

9. Use as claimed in any one of the preceding claims wherein the skin care composition is a sanitizing precursor comprising:
i. less than 15% by weight water;
ii. bipolar antimicrobial particle, quaternary ammonium antimicrobial agent or combinations thereof; and,
iii. a cationic surfactant;
wherein the sanitizing precursor is suitable to be diluted to produce a sanitizing composition comprising at least 80% by weight water.

10. Use as claimed in any one of the preceding claims wherein the skin care composition is a sanitizing composition comprising:
i. at least 80% by weight water;
ii. bipolar antimicrobial particle, quaternary ammonium antimicrobial agent or combination thereof; and
iii. a cationic surfactant.

11. Use according to any one of the preceding claims wherein the cationic surfactant in the sanitizing composition or precursor is selected from cetrimonium chloride, benzalkonium chloride, cetrimonium bromide or a mixture thereof.

12. Use according to any one of the preceding claims wherein the sanitizing composition is in a sprayable form or in a gel form or provided on a swab or provided on a sheet or a towelette.
